# EUROPEAN PATENT APPLICATION

(11) **EP 2 444 384 A1**
(43) Date of publication of application: **25.04.2012**
(21) Application number: 10788865.3
(22) Date of filing: 07.06.2010
(51) Int. Cl.: C07C 11/12, C07C 11/00, A24B 3/00, A24B 3/12, A24B 3/14, A24B 3/18, A24B 15/00, A24B 15/10, A24B 15/18, A24B 15/28, A24B 15/30, A24B 15/32

(54) **USE OF NEOPHYTADIENE AS ADDITIVE FOR LIQUID CIGARETTE**

(30) Priority: 19.06.2009 CN 200910012125
(71) Applicant: Li, Wenbo, Liaoning 110026 (CN)
(72) Inventor: Li, Wenbo, Liaoning 110026 (CN)
(74) Representative: Chung, Hsu Min
(86) International application number: PCT/CN2010/073621
(87) International publication number: WO 2010/145469

(57) **Abstract**

Neophytadiene used as additive for liquid cigarette can improve aroma and evaporation rate thereof. The liquid cigarette is composed of from 25 to 99.9% by weight percentage of atomized liquid and from 0.01 to 75% by weight percentage of neophytadiene.

## Description

### TECHNICAL FIELD

The present invention relates to evaporation in liquid cigarettes or additive for atomizing cigarettes, and particularly to applications of neophytadiene as an additive for liquid cigarettes.

### BACKGROUND

Presently, two kinds of sucking devices are known as cigarette substitutes and being useful for smoking cessation. One of them includes a porous body having a relatively large surface area retaining a liquid mixture of various components, which has a smell of cigarette. When inhaled air flows through the porous body, a good deal of liquid is vaporized and mixed with the air so as to provide satisfaction to the smoker; the other kind of sucking device places the liquid in an atomizing electronic cigarette, which vaporizes the liquid by an atomizer and provide the generated vapor to the user, so as to simulate functions of a regular cigarette. However, an important disadvantage of such devices is that they cannot simulate the taste and smell of regular cigarettes.

### SUMMARY OF THE INVENTION

To overcome the disadvantage of the arts, the present invention discloses an application of neophytadiene as an additive for liquid cigarettes.

In order to achieve the objects above, in a technology according to the present invention:

Neophytadiene is used as an additive for liquid cigarettes. Neophytadiene is a diterpenoid compound and is liquid at room temperature. The molecular weight of the neophytadiene is 278.52, its molecular formula is C₂₀H₃₈, and its structural formula is:

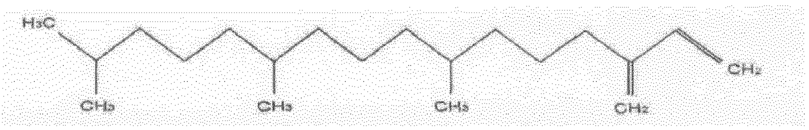

Such a liquid cigarette contains atomizing liquid and neophytadiene, specifically, 25∼99.9% weight percent atomizing liquid and 0.01∼75% weight percent neophytadiene. The atomizing liquid contains 60∼95% weight percent propylene glycol, 1∼30% weight percent glycerin, the rest contains flavoring essence, water and ethanol. The 0.01∼20% addition of neophytadiene can generate smell of a cigarette. The 20∼75% addition of neophytadiene can speed up the atomization.

Advantages of the present invention include:

1. In the present invention, neophytadiene is used as an additive for liquid cigarettes, so as to improve the taste of the inhaled gas or vapor, to make them have a smell of a regular cigarette.

2. In the present invention, the addition of neophytadiene facilitates the transformation of the liquid into gas, especially the vaporization in a liquid cigarette, by speeding up the atomization, so as to save electric power for batteries.

3. The 0.01∼20% neophytadiene addition in liquid cigarettes can improve the taste of the inhaled gas or vapor to have a smell of regular cigarettes; 20∼75% neophytadiene addition can speed up the volatilization of the liquid into gas.

Neophytadiene exists widely in tobaccos, and is one of the components of cigarette combustion smoke. Neophytadiene is almost odorless and has a smell of cerolipoid which is adverse to the smell of the smoke. Neophytadiene is a diterpenoid compound and is liquid at room temperature. The molecular weight of the neophytadiene is 278.52, and its molecular formula is C₂₀H₃₈.

### DETAIL EMBODIMENT OF THE INVENTION

Neophytadiene can be extracted from tobacco leaves, tobacco stems and wastes, or generated by dehydration of phytol.

Specifically, a method for preparing neophytadiene:

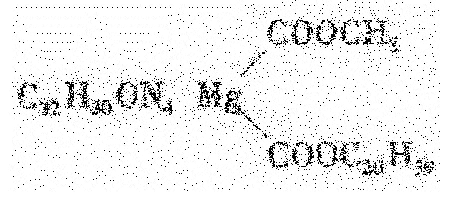

Chlorophyll:

A Chlorophyll molecule comprises two parts, a polar porphyrin head (N has a part of negative charges, Mg has a part of positive charges) and an apolar phytol tail.

Chlorophyll is hydrolyzed under an alkaline condition to generate phytol (C₂₀H₃₉OH):

C₅₅H₇₂O₅N₄Mg+2KOH -> CH₃OH+C₂₀H₃₉OH+C₃₄H₃₀O₅N₄MgK₂

Under the catalytic action and dehydration action of concentrated sulfuric acid, phytol is heated to 170°C, an elimination reaction happens and then generates neophytadiene (C₂₀H₃₈).

The generated neophytadiene is used as an additive for liquid cigarettes. It is formed by adding 0.01∼75% neophytadiene into conventional atomizing liquid; the conventional liquid typically contains 60∼95% weight percent propylene glycol, 1∼30% weight percent glycerin, the rest contains flavoring essence, water and ethanol.

The liquid cigarette contains atomizing liquid and neophytadiene, specifically, 25∼99.9% weight percent atomizing liquid and 0.01∼75% weight percent neophytadiene.

The 0.01∼20% neophytadiene addition in liquid cigarettes can improve the taste of the inhaled gas or vapor to have a smell of regular cigarettes.

The 20∼75% neophytadiene addition can speed up the volatilization of the liquid into gas.

Embodiment 2

Adding 0.01∼3% weight percent neophytadiene into conventional atomizing liquid as a liquid cigarette additive can generate smell of cigarettes, and improve the taste of inhaled gas or vapor, so as to provide it with smell of regular cigarettes.

Adding 20∼75% weight percent neophytadiene into conventional atomizing liquid as a liquid cigarette additive can speed up the vaporization of the liquid, increase the speed of the liquid's volatilization to become gas.

Embodiment 3

Adding 3∼10% weight percent neophytadiene into conventional atomizing liquid as a liquid cigarette additive can generate smell of cigarettes, and improve the taste of inhaled gas or vapor, so as to provide it with smell of regular cigarettes.

Adding 50∼70% weight percent neophytadiene into conventional atomizing liquid as a liquid cigarette additive can speed up the vaporization of the liquid, increase the speed of the liquid's volatilization to become gas.

## Claims

1. An application of neophytadiene as an additive for a liquid cigarette.

2. The application of neophytadiene as an additive for liquid cigarettes of claim 1, **characterized in that**, the neophytadiene is a diterpenoid compound and is liquid at room temperature, the molecular weight of the neophytadiene is 278.52, the molecular formula for the neophytadiene is C₂₀H₃₈, and the structural formula of the neophytadiene is:

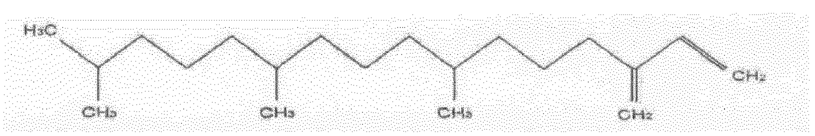

3. The application of neophytadiene as an additive for liquid cigarettes of claim 1, **characterized in that**: the liquid cigarette comprises atomizing liquid and neophytadiene, and specifically 25∼99.9% weight percent atomizing liquid and 0.01∼75% weight percent neophytadiene.

4. The application of neophytadiene as an additive for liquid cigarettes of claim 3, **characterized in that**: the atomizing liquid contains 60∼95% weight percent propylene glycol, 1∼30% weight percent glycerin, and the rest contains flavoring essence, water and ethanol.

5. The application of neophytadiene as an additive for liquid cigarettes of claim 3, **characterized in that**: an addition of 0.01∼20% neophytadiene generates smell of cigarettes.

6. The application of neophytadiene as an additive for liquid cigarettes of claim 3, **characterized in that**: an addition of 20∼75% neophytadiene increases a speed of vaporization.
